# EUROPEAN PATENT APPLICATION

(11) **EP 0 545 343 A1**
(43) Date of publication of application: **09.06.1993**
(21) Application number: 92120425.1
(22) Date of filing: 30.11.1992
(51) Int. Cl.: C12N 15/12, C12N 15/62, C07K 13/00

(54) **Ligand-binding domains of fibroblast growth factor receptors and chimeric proteins containing these domains**

(30) Priority: 02.12.1991 IL 100219
(71) Applicant: YEDA RESEARCH AND DEVELOPMENT CO. LTD., Rehovot 76100 (IL)
(72) Inventor: Yayon, Avner, Meshek 104, 73272 (IL); Givol, David, St. Rehovot 86300 (IL)
(74) Representative: Grünecker, Kinkeldey, Stockmair & Schwanhäusser Anwaltssozietät

(57) **Abstract**

Regions within the extracellular portions of a fibroblast growth factor receptor (FGFR) confer ligand specificity of the receptor. One such ligand specificity region (LSR) is included in the C-terminal region of the third immunoglobulin like domain of the FGFR and another in the second immunoglobulin like domain. By removing the LSR from an FGFR and replacing it with a corresponding LSR from another FGFR, a chimeric protein is obtained which can bind a ligand with a specificity of binding being determined by the inserted LSR.

## Description

### FIELD OF THE INVENTION

The present invention is in the field of fibroblast growth factor receptors (FGFR) and more specifically concerns regions within the extracellular portion of FGFR which confer the ligand binding specificity of the receptor. The region conferring ligand binding specificity of such receptors will be referred to herein as "ligand specificity region (LSR)".

The present invention also concerns chimeric proteins comprising an extracellular portion of an FGFR with at least one of its LSR removed and replaced by that of another FGFR. It was found in accordance with the present invention that the binding specificity of such a chimeric protein is determined by the LSR.

The present invention further concerns DNA molecules coding for said LSR and said chimeric proteins, vectors comprising said DNA molecules, cells transfected by said vectors and the production of a recombinant expression vector therefrom.

### PRIOR ART

The following is a list of prior art believed to be pertinent to the present invention:
1. Lee, P.L., Johnson, D.E., Cousens, L.S., Fried, V.A. & Williams, L.T. Science, 245, 57-60 (1989).
2. Ruta, M., Howk, R., Ricca, G., Drohan, W., Zabelshansky, M., Laurey, G., Barton, D.E., Francke, U., Schlessinger, J & Givol, D. Oncogene 3, 9-15 (1988).
3. Kornbluth, S., Paulson, K.E. & Hanafusa, H. Mol. Cell Biol. 8, 5541-5544 (1988).
4. Dionne, C.A., Crumley, G., Bellot, F., Kaplow, J.M., Searfoss, G., Ruta, M., Burgess, W.H., Jaye, M. & Schlessinger, J. EMBO J 9, 2685-2692 (1990).
5. Partanen, J., Mäkelä, T., Eerola, E., Korhonen, J., Hirvonen, H., Claesson-Welsh, L. & Alitalo, K. EMBO J. 10, 1347-1354 (1991).
6. Miki, T., Fleming, T.P., Bottaro, D.P., Rubin, J.S., Ron, D. & Aaronson, S. Science 251, 72-75 (1991).
7. Pasquale, E.B. Proc. Natl. Acad. Sci. USA 87, 5812-5816 (1990).
8. Avivi, A., Zimmer, Y., Yayon, A., Yarden, Y. & Givol, D. Oncogene 6, 1089-1092 (1991).
9. Keegan, K., Johnson, D.E., Williams, L.T. & Hayman, M.J. Proc. Natl. Acad. Sci. USA 88, 1095-1099 (1991).
10. Berger, J., Hauber, J., Hauber, R. & Cullen, B.R. Gene 66, 1-10 (1988).
11. Mansukhani, A., Moscatelli, D., Talarier, D., Levytska, V & Basilico, C. Proc. Natl. Acad. Sci. USA 87, 4378-4382 (1990).
12. Johnson, D.E., Lee, P.L., Lu, J. & Williams, L.T. Molec. Cell Biol 10, 4728-4736 (1990).

In the following description reference to the above publications will be made by indicating their number from the above list.

### BACKGROUND OF THE INVENTION

Binding of cellular growth factors to their receptors constitutes a highly specific interaction and the basis for cell and tissue-type specific growth and differentiation. The fibroblast growth factors (FGF) family consists of at least seven closely related polypeptides involved in the control of cell growth, embryonal development, angiogenesis and malignant transformation. The FGFs show a variety of biological activities towards cells of mesenchymal, neuronal and epithelial origin. At least five FGF receptors (FGFRs) have recently been identified and cloned¹⁻⁹. All FGFRs are receptor tyrosine kinases and they share a common basic structure of three extracellular Immunoglobulin-like (Ig) domains and a split kinase as their intracellular catalytic domain, which is activated upon ligand binding.

Further, the FGFRs have ligand binding profiles which suggest a certain degree of cross-reactivity, for example, acidic FGF (aFGF), basic FGF (bFGF) and hst/Kfgf (or FGF4) are capable of binding to bFGFR, also known as Bek or FGFR2, encoded by the murine gene bek⁴. The keratinocyte growth factor (KGF), a member of the FGF family, however, shows a high degree of specificity as it binds solely to the keratinocyte growth factor (KGFR), also encoded by the murine gene bek. KGFR, besides being capable of binding KGF is also capable of binding aFGF and FGF4, but not bFGF. Another FGFR, FGFR1, encoded by the murine gene flg is similar to FGFR2 as it is also capable of binding aFGF, bFGF and FGF4 and incapable of binding KGF^{4,11,12}.

To date there is no clear evidence concerning the precise molecular basis for the ligand and tissue specificities of the various FGFRs, nor for the above-noted observed variation in ligand specificity between various FGFRs.

In accordance with the present invention, the molecular basis for the ligand specificity has been elucidated and regions conferring ligand specificity have been identified. One such region was found to be included in the 50 amino acids long C-terminal half of the third immunoglobulin-like (Ig) domain (hereinafter "Ig-domain 3") of the extracellular portion of FGFR. Another such region was found to be located in the second Ig-like domain (hereinafter "Ig-domain 2").

### OBJECT OF THE INVENTION

It is an object of the present invention to provide a ligand specificity region (LSR) of the extracellular portion of FGFR, i.e. a region of the extracellular portion which confers ligand-binding specificity.

It is another object of the present invention to provide a soluble chimeric protein which comprises an extracellular portion of one kind of FGFR in which an LSR was replaced by a corresponding LSR from another FGFR.

It is a further object of the present invention to provide DNA molecules encoding an LSR or said chimeric protein.

Further objects of the invention will be elucidated in the following description.

### SUMMARY OF THE INVENTION

In accordance with the present invention it has been found that the ligand specificity of the FGFR is conferred by two regions in the extracellular portion of FGFR, such a region referred to herein as "LSR" (ligand specificity region). One of these regions was found to be situated at the C-terminal half of the immunoglobulin-like domain 3 ("Ig-domain 3") of the extracellular portion of the FGFR and the other was found to be included in the second immunoglobulin like domain ("Ig-domain 2") thereof. Chimeric proteins comprising regions from the extracellular portion of an FGFR which do not confer ligand binding specificity ("non-LSR") from one FGFR and an LSR (or a plurality of such regions - LSRs) from another FGFR have been prepared, which had binding specificity similar to that of the FGFR from where said LSR originated.

In accordance with the invention DNA molecules encoding said LSRs or encoding the chimeric proteins have been constructed, expression vectors comprising them have been prepared, cells were transfected by these vectors and recombinant expression products were obtained therefrom.

In accordance with a first of its aspects, the present invention provides a polypeptide being a member selected from the group consisting of:
(a) a polypeptide having an amino acid sequence of the ligand specificity region (LSR) from the extracellular portion of an FGFR, which region confers the ligand binding specificity of said FGFR;
(b) a polypeptide having the amino acid sequence of the polypeptide of (a) in which one or more amino acids residues has been added, replaced or deleted, which polypeptide when inserted into an extracellular portion of an FGFR in place of said LSR, confer upon said FGFR the same ligand binding specificity of said LSR; and
(c) a recombinant polypeptide or protein having an amino acid sequence comprising the amino acid sequence of (a) or (b).

The above polypeptide may comprise the LSR from the C-terminal region of Ig-domain 3 or the LSR from Ig-domain 2. As will be appreciated by the artisan it is possible, without undue experimentation, to prepare such polypeptides in which the LSR has been modified by adding, replacing or deleting an amino acid residue without amending its ligand specificity properties. This may be achieved, for example, by mutating a cDNA coding for such an LSR in a controlled manner, and cloning the mutated cDNA and then checking the product of this cloned DNA for its ligand specificity properties, e.g. by inserting it into a chimeric protein (see below).

In accordance with a second of its aspects, the present invention provides a chimeric protein comprising one or more first regions derived from a first protein and one or more second regions derived from a second protein, said first and second regions being fused to one another; the chimeric protein being characterized in that:
(i) said one or more first regions being each independently a member selected from the group consisting of -
   (a) a polypeptide chain having an amino acid sequence of a first LSR from the extracellular portion of a first FGFR, and
   (b) a polypeptide chain having the ligand specificity properties of said first LSR and having the amino acid sequence of the polypeptide in (a) in which one or more amino acid residues has been added, replaced or deleted;
(ii) said one or more second regions being each independently a member selected from the group consisting of -
   (a) a polypeptide chain having an amino acid sequence of a region of a second FGFR which is not the LSR region of said second FGFR (non-LSR), and
   (b) a polypeptide chain having the amino acid sequence of said non-LSR wherein one or more amino acid residues has been replaced or added without essentially affecting the biological properties of said one or more second regions or binding specificity of said first LSR; and in that
(iii) said chimeric protein being capable of binding a ligand and having a ligand binding specificity conferred by said one or more first regions.

Such a chimeric protein may comprise the entire non-LSR from the second FGFR or only a portion thereof. Typically, however, the chimeric protein will comprise a major part of the extracellular portion of the second FGFR wherein one or both of its LSRs has been replaced by one or both corresponding LSRs from one or more other FGFRs or by such LSRs in which one or more amino acid residue has been added, replaced or deleted without affecting the ligand specificity properties thereof.

Thus, the chimeric protein of the invention may comprise fused regions from two different FGFRs, i.e. one or two LSRs coming from one FGFR and the non-LSR coming from another FGFR; or may comprise fused regions from a plurality of FGFRs, e.g. one LSR coming from one FGFR, another LSR coming from another FGFR and the non-LSR coming from a third FGFR or even the non-LSRs of such an FGFR may come from a plurality of FGFRs.

By a third of its aspects, the present invention provides a DNA molecule encoding the above polypeptide or chimeric protein. The DNA molecule of the invention comprises a coding sequence being a member selected from the group consisting of:
(a) a nucleotide sequence encoding the ligand specificity region (LSR) from the extracellular portion of an FGFR which region confers ligand binding specificity of said FGFR;
(b) a nucleotide sequence of (a) in which one or more codons has been added, replaced or deleted, provided that the encoded polypeptide retains the same ligand specificity properties of said LSR;
(c) a recombinant nucleotide sequence encoding a polypeptide and comprising the nucleotide sequence of (a) or (b).

A particular example of the DNA molecule of the invention is one which encodes a chimeric protein, in which case the DNA molecule comprises in addition to a nucleotide sequence of (a) or (b), also one or more other sequences fused thereto which encode amino acid sequences derived from non-LSR from another FGFR.

The present invention still further provides expression vectors comprising the above DNA molecules and cells transfected by said expression vectors.

Still further, the present invention provides a recombinant DNA method for preparing the above chimeric FGFR which comprises transfecting a host cell by said vector and recovering an expression product of said vector from the transfected cell.

If desired, antibodies directed against a specific LSR may be prepared by immunizing an animal with a peptide or chimeric protein including the specific LSR, and then screening the antibodies with various soluble portions of FGFR and various fused proteins, e.g differential screening with polypeptides or chimeric proteins comprising the specific LSR or comprise another LSR on the other hand, until a specific antibody is obtained.

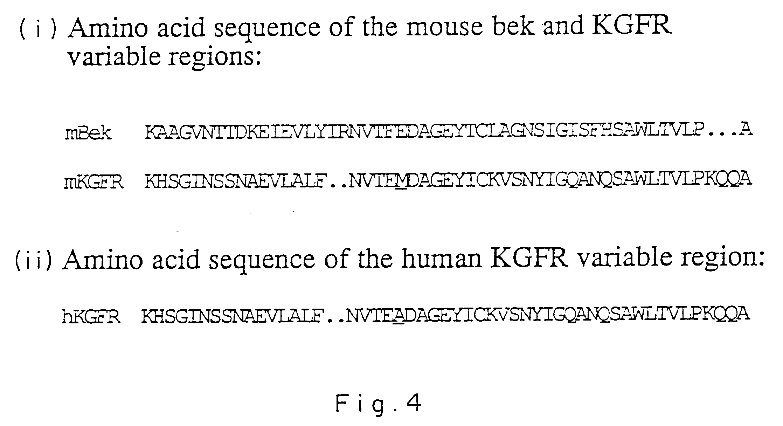

In accordance with the present invention experiments conducted with a number of FGFRs including the murine FGFR1, FGFR2 and KGFR, and the human K-Sam. These FGFRs were found to contain LSRs, and replacing an LSR of one FGFR with that of another, was found to change ligand binding specificity of the resulting chimeric protein. However, although experiments reported hereinbelow are limited to those aforementioned four KGFRs, it will be clear to the artisan that this does not limit the present invention only to these four receptors. On the contrary, by the knowledge gained in accordance with the present invention, the artisan will have no difficulties in finding LSRs in other FGFRs based on the knowledge of their location gained in accordance with the present invention, and prepare chimeric proteins using LSRs and non-LSRs from such other FGFRs. Furthermore, based on a high degree of homology between similar FGFRs from different mammals (see for example Fig. 4), by taking, for example, a cDNA encoding one LSR, and by means of hybridization tests under standard conditions of stringency, one may easily obtain LSRs from other species.

The LSR, and specifically the one at the C-terminal region of the Ig-domain 3, designated herein at times as the "variable region", and a chimeric protein comprising an extracellular portion of an FGFR wherein one or both of its LSRs, specifically a variable region, has been replaced with a corresponding LSR from another FGFR, may have important potential therapeutic use, e.g as a chemo-therapeutic agent in various malignancies associated with increased production by cells of FGFR. Such LSR and chimeric proteins may competitively bind the ligand of the overly produced FGFR and accordingly inhibit proliferation of the tumor cells. Furthermore, a peptide comprising the LSR or a chimeric protein may also possibly have various diagnostic applications, such as in quantitatively determining the amount of ligand in a medium.

The chimeric proteins are particularly useful for the above purposes since they may be tailored to have various ligand specificity profiles.

### BRIEF DESCRIPTION OF THE DRAWINGS

**Fig. 1** is a schematic illustration of the structure and sequence of the murine FGFR2 (Bek) and KGFR, and a comparison between them as described in Example 1;
**Fig. 2** is a schematic illustration of the construction of FGFR2 and chimeric KGFR/FGFR2 in the alkaline phosphatase expression vector (APtag) as described in Example 1;
**Fig. 3** illustrates the DNA sequence of the PCR product obtained from mouse skin RNA as described in Example 1;
**Fig. 4** illustrates the amino acid sequences of (i) the mouse FGFR2 and KGFR variable regions and (ii) the human KGFR variable region, as described in Example 1;
**Fig. 5** is a bar-graph representation of the ligand binding profiles (a-acidic FGF, b-basic FGF and k-KGF) of secreted soluble FGFR2 (BAP) and FGFR2/KGFR (K-BAP) receptors, as described in Example 1;
**Figs. 6A and 6B** are graphic representations of the competitive binding analysis of the binding of ligand (FGFRs) to soluble FGF receptors, as described in Example 1;
**Fig. 7** is a representation of the exposed X-ray film showing the results of a competitive binding between soluble, secreted FGFR2 (BAP) and FGFR2/KGFR (K-BAP) receptors to their ligands in the presence of a cold ligand legend (b-basic FGF, a-acidic FGF, k-KGF), as described in Example 1;
**Fig. 8** is a schematic representation of the cDNA of the ectodomains of the receptors FGFR1 and KGFR and the chimeric receptors FGFR1/KGFR (F1/K) and FGFR1/FGFR2/KGFR (F1/F2/K), as described in Example 2;
**Fig. 9** is a bar-graph representation of the ligand binding profile of the soluble secreted receptors FGFR1 and KGFR and chimeric receptors FGFR1/KGFR (F1/K) and FGFR1/FGFR2/KGFR (F1/F2/K), as described in Example 2;
**Fig. 10** is a graphic representation of the displacement analysis results of the binding of various FGFs to soluble secreted receptors FGFR1 and KGFR and the soluble secreted chimeric receptor FGFR1/KGFR, as described in Example 2;
**Fig. 11** is a graphic representation of the displacement analysis results of the binding of various FGFs to soluble secreted receptors FGFR1 and KGFR and the soluble secreted chimeric receptor KGFR1/FGFR2/KGFR, as described in Example 2; and
**Fig. 12** is a presentation of a number of reproductions of the exposed X-ray films showing the results of cross-linking between soluble secreted FGFR1 and KGFR receptors and chimeric FGFR1/KGFR and KGFR1/FGFR2/KGFR receptors to various ligands (FGFs) as described in Example 2.

In the following non-limiting examples is a description of specific embodiments of the invention in which reference is made to the above-noted accompanying Figures.

### EXAMPLE 1

To address the question of ligand specificity within the two large families of FGF ligands and FGF receptors, genetic engineering techniques were employed to substitute discrete regions between two closely related FGF receptors. In the following results there is demonstrated that a confined, 50 amino acids long, variable region within the 3rd immunoglobulin-like domain of the murine Bek (FGFR2) and the keratinocyte growth factor receptor (KGFR), exclusively determines their ligand binding specificities. Moreover, as arises from the following results, the carboxy terminal half of the 3rd immunoglobulin-like domain of the FGF receptors, "variable region", is a major determinant for ligand binding, and appears to be involved in a novel mechanism for alternating receptor-ligand specificity and generating receptor diversity.

To study the possible contribution of the variable half of the 3rd Ig domain to the binding specificity of FGFR2, a chimeric molecule between FGFR2 and KGFR was generated.

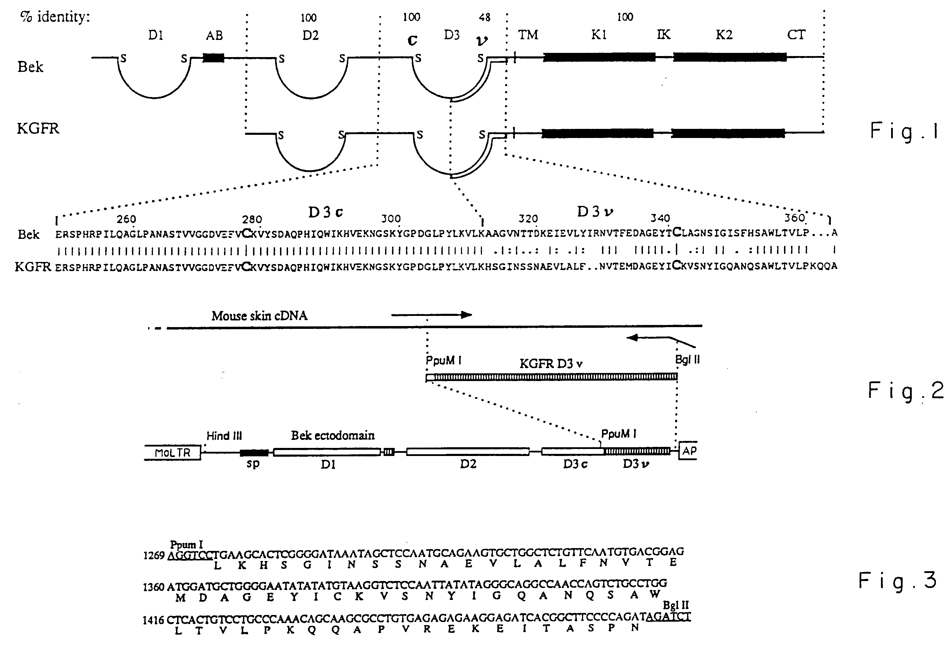

In Fig. 1 there is illustrated schematically, the structure and sequence of the murine FGFR2 and KGFR and a comparison between them. The overall structure of the two receptors is shown as is the amino acid sequence identity. The designations: D1, D2 and D3 represent the Ig-like domains; AB represents the acidic box; TM represents the transmembrane region; K1 and K2 represent the tyrosine kinase regions; IK represents the interkinase segment; and CT represents the C-terminal tail. The amino acid sequence, presented in the form of a one-letter code, depicts the differences in D3 between FGFR2 and KGFR. The N-terminal region containing the constant sequence is denoted ***c*** and the C-terminal region containing the variable sequence is denoted ***v***. As shown in Fig. 1, FGFR2 possesses a typical 3 Ig domain structure, while the KGFR lacks both the first Ig domain and the conserved acidic box⁶. In addition it was noticed that a restricted variable sequence (*v*) was present at the carboxy-terminal half of the 3rd Ig domain in which there is only 48% sequence identity between the two receptors, compared with 100% identity in the constant (*c*) sub-domain.

Fig. 2 illustrates the construction of FGFR2 and chimeric KGFR/FGFR2 in the alkaline phosphatase expression vector (APtag)^{10,11}. To prepare the extracellular portion of FGFR2 in APtag, the FGFR2 clone in vector pBS (Bluescript™) was subjected to standard polymerase chain reaction (PCR) methods using the following primers:
1) 5'-GAAGCTTACCGTCCACGTGG corresponding to the sequence beginning 50bp upstream to the ATG and containing a HindIII site, and
2) 5'-TCGCGAAGATCTATCTGGGGAAGCCGT corresponding to the sequence ending two codons 5' to the transmembrane region and containing a BgIII site.
   The reaction mixture (100µl) was a solution of 67 mM Tris-HCl, pH 8.8, 6.7 mM MgCl₂, 16.6 mM (NH₄)₂ SO₄, 250 mM dNTP, 0.17 mg/ml bovine serum albumin, 0.5 nM of each primer and 5 units (u) of Taq polymerase (Promega). After 30 cycles of 1.5 min at 94°, 1.5 min at 65°, and 4 min at 72° the reaction product was extracted with phenol and phenol-chloroform (1:1) and precipitated with ethanol. The DNA was dissolved and, following site-filling with dNTP and Klenow enzyme, was cloned into the EcoRV sites of pBS. The HindIII-BgIII fragment from such a clone encoding the extracellular region of Bek was isolated and cloned into the HindIII and BgIII digested APtag vector.
   To prepare the chimeric KGFR/FGFR2, a cDNA corresponding to the KGFR-v region of D3 was cloned from mouse skin RNA as follows: cDNA was prepared from total mouse skin RNA by incubating 10µg RNA in 20 µl of 50 mM Tris-HCl pH 8.15, 6 mM MgCl₂, 110 mM KCl, 1 mM dithiothreitol, 20µg/ml RNAasin, 250 M dNTP and 1 nM primer 2 with 10 u of AMV reverse transcriptase (Genetic Resources) at 42° for 1 hr. Half of the reaction mixture (10µl) was diluted to 100µl by PCR buffer (see above) containing 0.5 nM of primer 2) and 0.5 nM of the following primer:
3) 5'-AAGGTCCTGAAGCACTCGGGGATA which overlaps the constant region and the KGFR variable region of D3 containing the PpuM I site.

After the addition of 5u of Taq polymerase, PCR was carried out for 35 cycles as described above. The DNA was treated with Klenow enzyme and dNTP, and the PCR fragment (230 bp) was isolated by electrophoresis on agarose gel, cloned into the EcoRV site of pBS and analyzed by DNA sequencing. The DNA sequence of this KGFR-v region of D3 obtained from mouse skin is schematically illustrated in Fig. 3. The DNA sequencing was performed using the standard chain termination procedure. The PpuMI-BgIII fragment from this clone, the HindIII-PpuMI fragment of FGFR2 (see Fig. 1) and the HindIII-BgIII digested APtag were all co-ligated to generate the chimeric receptor depicted in Fig. 2.

Thus, the above procedure for generating a chimeric molecule between FGFR2 and KGFR may be summarized as follows: Mouse skin RNA was used to prepare cDNA which was amplified by PCR using two oligonucleotides flanking the variable half of KGFR and overlapping the common half of domain 3, as illustrated in Fig. 2. The shared restriction sites were used for replacing the bek (FGFR2 encoding gene) variable segment with the obtained PCR product. Sequence analysis of the PCR product and the chimeric construct confirmed their identity to the KGFR sequence in that region⁶, and the in-frame insertion of the variable segment, as illustrated in Fig. 3.

To determine the ligand-binding profiles of FGFR2 and the chimeric receptor independently, the APtag expression vector^{10,11} was used to express the extracellular portion of the receptor as a secreted alkaline-phosphatase (AP) fused protein (see illustration in Fig. 2). Both FGFR2-AP (also called BAP, and in short form F2-AP, hereinafter will be called either soluble FGFR2 or F2) and the chimeric KGFR/FGFR2-AP (also called K-BAP or K-F2-AP, hereinafter will be called soluble chimeric FGFR2/KGFR or F2/K) constructs were transfected into NIH 3T3 cells and receptor secreting clones were selected by the determination of AP activity in the conditioned medium of the transfected cell cultures.

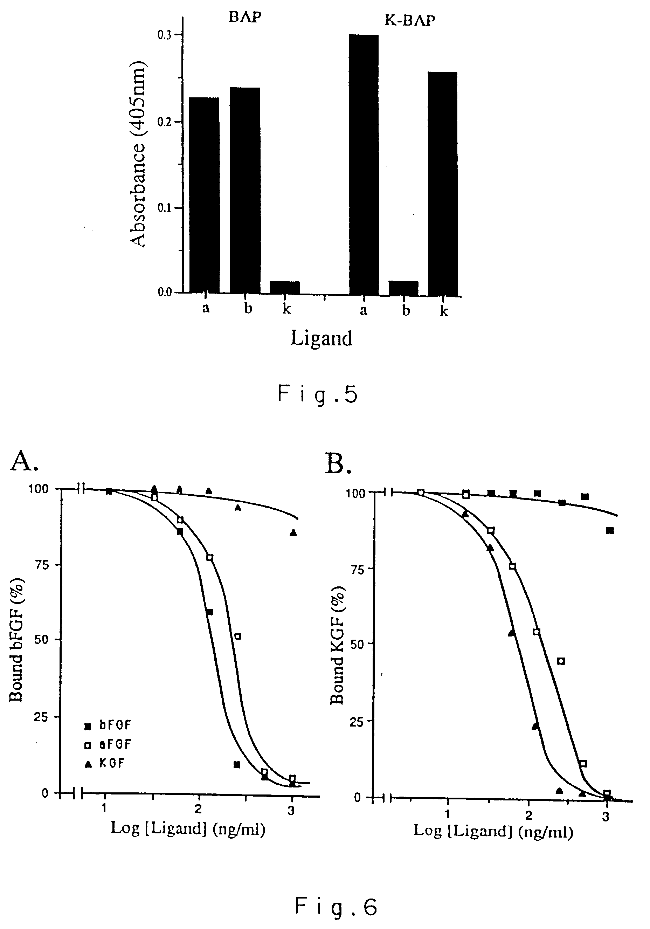

Fig. 5 illustrates, schematically, the ligand binding profiles of secreted soluble FGFR2 and FGFR2/KGFR. These ligand binding profiles were obtained by performing an assay which makes use of heparin Sepharose-bound ligands (FGFs) to bind receptor-AP fusion proteins, the ligand binding being determined by measuring the alkaline phosphatase (AP) activity on the conjugated beads. Immobilized ligand was prepared by incubation of 300µl of heparin-Sepharose slurry (Pharmacia) with 1µg of either human recombinant KGF (Amgen), human recombinant bFGF (Takeda, Japan) or human recombinant aFGF (obtained from Dr. G. Neufeld Weizmann Institute). After 30 min shaking at room temperature the beads were washed 3 times with 0.5 M NaCl and suspended (1:1) in 0.01M phosphate buffer pH 7.4, 0.15 M NaCl (PBS). For receptor binding analysis, 30µl of ligand-heparin Sepharose beads were incubated with 10µl to 15µl of medium from either confluent FGFR2 secreting cells or FGFR2/KGFR secreting cells, normalized to total AP activity. After 30 min of shaking at room temperature the beads were washed 3 times with 0.5 M NaCl, once with 10 mM Tris-HCl pH 8.0 and incubated at 65° for 10 min to inactivate endogenous cellular AP. The beads were assayed for AP, by measuring absorbance at 405 nm after 15 min of incubation with 1.0 M diethanolamine (pH 9.8), 0.5 mM MgCl₂, 10 mM L-homoarginine, 0.5 mg/ml bovine serum albumin (Sigma) and 12 mM p-nitrophenylphosphate. FGFR2 and FGFR2/KGFR corresponds to the secreted receptors derived from cells transfected with FGFR2 or FGFR2/KGFR respectively. The designations a, b, k in Fig. 5 represent heparin-Sepharose bound acidic FGF, basic FGF and KGF, respectively.

As shown in Fig. 5, the soluble FGFR2 retains its ligand binding and specificity, namely, the soluble FGFR2 was found to bind aFGF, bFGF and also hst/Kfgf (also called FGF4 as used hereinafter, results not shown), but not KGF, in agreement with studies on cells which overexpress either bek or flg⁴. The FGFR2/KGFR chimera, however, acquired a different binding profile compared to the parental FGFR2 as it lost the capacity to bind bFGF and gained full capacity to bind KGF. The acquired ligand binding profile of FGFR2/KGFR is very similar to that reported for the cellular KGFR.

Displacement binding analysis of FGFR2 and FGFR2/KGFR with the three ligands was performed in the presence of heparin and using immobilized anti-AP antibody to separate receptor bound-ligand. None of the ligands bound to any of the FGF receptors studied in the absence of heparin in agreement with the previously described absolute heparin-dependent binding of bFGF.

Fig. 6A is a graphical illustration of the binding of aFGF, bFGF and KGF to soluble FGFR2 receptors, in a competitive binding assay. The reaction mixture (200µl) contained 2 ng of ¹²⁵I-labelled bFGF, increasing amounts of unlabelled ligand, 0.2µg heparin (Hepar, Franklin Ohio) 0.2% BSA, 25 mM Hepes, pH 7.4, and 100µl of conditioned medium from cells secreting BAP. After 30 min of incubation at room temperature, 15µl of agarose-protein A, prebound to rabbit anti-human placental AP antibodies, was added and the reaction mixture was shaken for 30 min at room temperature. The beads were washed twice with HNTG (20 mM Hepes pH 7.5 mM NaCl, 1% Triton x-100 and 10% glycerol) and once with 0.5 M NaCl to eliminate non-specifically bound ligands, and the radioactivity associated with the beads was then counted in a γ-counter.

Fig. 6B is a graphical illustration of the binding of aFGF, bFGF and KGF to soluble FGFR2/KGFR receptors, in a competitive binding assay, the assay conditions being as noted above except that the labelled ligand was ¹²⁵I-KGF and the conditioned medium was from cells secreting FGFR2/KGFR. For both above ¹²⁵I-labelled ligands, iodination was performed on 2µg ligand by the lactoperoxidase method, and purification on heparin-sepharose. Specific activity of the various labelled ligands was between 150,000-250,000 cpm/ng.

Thus, the results shown in Fig. 6A and 6B show that the soluble receptor FGFR2 binds aFGF and bFGF with a dissociation constant of 7nM and 9nM respectively, and does not bind KGF. Furthermore, the FGFR2/KGFR receptor binds aFGF and KGF with a dissociation constant of 8nM and 5nM respectively and with no detectable binding of bFGF under the same experimental conditions. Both FGFR2 and FGFR2/KGFR bind aFGF with a similar affinity resembling the ability of aFGF to bind to FGFR2 and KGFR expressing cells. The above competition binding experiments not only confirmed the unique ligand profile of the chimeric receptor but further suggest that aFGF, bFGF and KGF all compete on the same ligand binding site. The affinity of the different ligands to either FGFR2 or FGFR2/KGFR was 10 to 30 fold lower than that reported for the respective integral membrane receptors⁶.

Chemical cross-linking of the three ligands to the parental and chimeric receptors further demonstrated the exclusive contribution of the variable segment to ligand specificity.

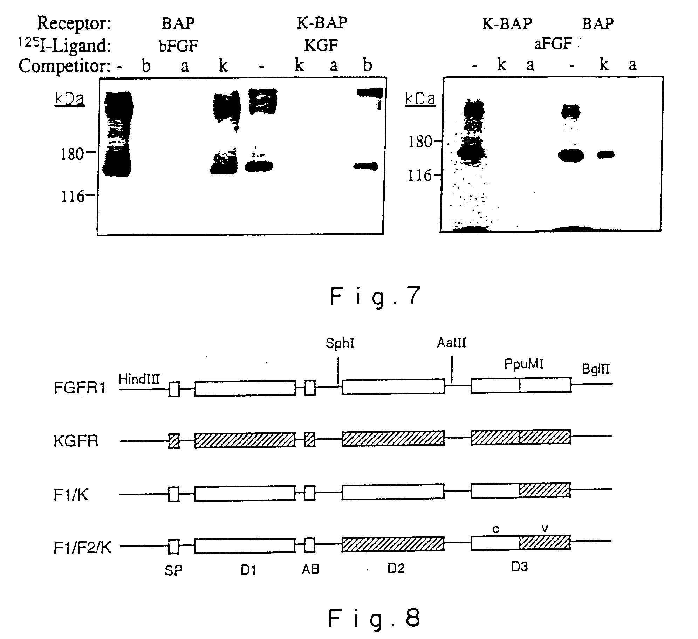

Fig. 7 shows the results of the cross-linking of soluble secreted FGFR2 and FGFR2/KGFR to FGFs. Conditioned medium from confluent cells secreting either FGFR2 or FGFR2/KGFR (100 µl) was reacted with either ¹²⁵I-KGF, ¹²⁵I-aFGF or ¹²⁵I-bFGF (10 ng/ml) in the presence of 1 ug/ml heparin and in the absence or presence of 100-fold cold (unlabelled) ligand followed by immunoprecipitation with Agarose-protein A-anti AP. After washing, the beads were suspended in 0.4 ml of 0.15 mM disuccinylimidyl suberate (DSS, Pierce) in PBS to effect the chemical cross-linking. After 30 min at room temperature the beads were spun and suspended in 1 ml of 150 mM glycine in 10 mM Tris-HCl pH 7.5 to terminate the cross-linking reaction. Five min later the beads were washed with PBS, boiled for 5 min in sample buffer and the proteins analyzed by polyacrylamide gel electrophoresis (PAGE) on a 6% gel. After the run the gel was dried and exposed to an X-ray film for 16 h. A reproduction of the exposed X-ray film is shown in Fig. 7, in which the competitive ligands are designated b, c and k, representing bFGF, aFGF id KGF, respectively.

From the results shown in Fig. 7 it may be concluded that FGFR2/KGFR cross-linked to KGF and this cross-linking could be inhibited by an excess of either KGF or aFGF but not of bFGF. FGFR2 or FGFR2/KGFR could not be cross-linked to KGF or bFGF, respectively. It is of interest to note that while FGFR1 (Flg gene product) and FGFR2 (Bek gene product) naturally exist in either two or three Ig domain forms, which bind equally well bFGF¹¹, only two Ig domain forms of KGFR were found to date⁶. On the basis of the art available to date one could not rule out, therefore, that the first Ig domain may have a specific dominant negative effect on the binding of KGF to its receptor. The fact that the chimeric FGFR2/KGFR contains the amino terminal Ig domain, while demonstrating a ligand binding profile indistinguishable of KGFR, rules out that possibility. It can therefore be concluded that the substituted 50 amino acids long variable region at the C-terminal half of domain 3, determines receptor-ligand specificity.

On the basis of amino acid sequence comparison of the Ig domain 3 it may well be that K-SAM, an amplified gene in human stomach tumors, encodes the human FGFR homologue. Sequence variability in the same region of the h4-variant of human flg¹² suggests that such a mechanism of alternative exon may exist in other members of the FGFR family as well.

The variable portion of Ig domain 3 contributes, according to the present analysis, most of the necessary interactions of the binding of KGF. The difference in sequence between FGFR2 and KGFR (Fig. 1) can be assigned to two segments; one (C-terminal) corresponds to the complementarity determining region 3 (CDR3) of the Ig *v* region, whereas the other may partially correspond to CDR2. This may imply that the binding of FGF to their receptors is by interactions with two or more polypeptide loops protruding from the Ig-like framework of the domain similar to the interactions between antigen and antibody. These structural similarities may have important implications for future modelling of FGFR-ligand interactions and for the design of receptor antagonists.

Based on the data acquired from the mouse receptors the human counterparts may be tested. Based on these results it was identified that the above-noted K-Sam, an amplified gene in human stomach cancer fulfills the criteria as the human KGF receptor. Sequence comparison (see Fig. 4) of the identified variable regions of the murine KGFR and K-Sam reveal almost complete sequence identity and with a single amino acid substitution in that region.

### EXAMPLE 2

FGFR1 and FGFR2 bind aFGF and bFGF with similar affinity^{4,11}. Thus, to address the question whether the product of the KGFR variable exon, which is highly homologous to the variable exon of the C-terminal half of Ig domain 3 in the FGFR1 gene, will also confer KGF binding on this receptor, the above-mentioned (Example 1) receptor chimera approach was employed. In the following results there is demonstrated that the FGFR1/KGFR chimera (in which the KGFR denoted the variable region) retained the high affinity binding towards aFGF and FGF4 and lost the capacity to bind bFGF but, unexpectedly, only gained a partial capacity to bind KGF. High affinity binding of KGF was gained only when domain 2 of FGFR1 was replaced by the homologous region derived from FGFR2, to form a FGFR1/FGFR2/KGFR chimera.

Thus, both the above-noted (Example 1) FGFR2/KGFR chimera and the FGFR1/KGFR chimera similarly retained their binding affinity towards aFGF and FGF4 and lost the binding capacity towards bFGF, this being indicative that the sequence of the C-terminal half of the Ig domain 3 determines a major binding element towards bFGF, while at the same time, this sequence is not crucial for the binding of aFGF and FGF4. However, with respect to KGF binding, the failure of the sequence of the C-terminal half of Ig domain 3 from KGFR to confer high affinity for KGF when inserted into FGFR1, this in contrast to its full capacity to do so when inserted into FGFR2 (Example 1), is indicative that contributions from other regions of the FGFR are necessary to support high affinity KGF binding.

Since the FGFR1 and FGFR2 differ in multiple positions in their ectodomain sequences, it is therefore likely that differences in the N-terminal half of domain 3 or in domain 2 are responsible for the above-noted differential binding of KGF to the FGFR1/KGFR and FGFR2/KGFR chimeras, this likelihood being supported by the ability of the above-noted FGFR1/FGFR2/KGFR chimera to bind KGF with high affinity. The difference between the FGFR1/KGFR and FGFR1/FGFR2/KGFR chimeras is not in the sequence of the C-terminal half of domain 3, which remained as a FGFR1/KGFR chimeric sequence, but rather in the sequence of domain 2 wherein the FGFR1 sequence was replaced by the FGFR2 sequence.

Thus, non-contiguous regions from both Ig domains 2 and 3 of FGFRs contribute the major elements which define ligand binding specificity of FGFRs, this in view of the fact that it has been demonstrated that deletion of Ig domain 1 does not alter binding specificity or affinity^{11,12}.

To prepare the FGFR1/KGFR chimera, denoted F1/K in Fig. 8, a similar procedure was followed to that described above in Example 1 for the preparation of the FGFR2/KGFR chimera, as follows: the murine flg clone (Bluescript™) encoding FGFR1 was subjected to PCR (25) cycles using the following primers:
4) 5'-CGAGCTCAAGCTTGTGGAATATCCATGGAG, corresponding to the sequence beginning 40 bp upstream to the ATG and containing a HindIII site), and
5) 5'-GTCTTCAGGACCTGGACATAAGGC corresponding to the sequence in the FGFR1 protein encoding amino acids 344-351).

The second primer contains a T/C substitution (underlined above) at nucleotide 1042 (which changed IIe 348 to Val) to generate the PpumI site, in homologous position to the PpuMI site in KGFR cDNA (see Example 1). The reaction mixture (100 µl) was 67 mM Tris HCl pH 8.8, 6.7 mM MgCl₂, 16.6 mM (NH₄)₂SO₄, 250 mM dNTP, 0.17 mg/ml BSA, 0.5 mM of each primer and 5 u Taq polymerase (Promega). After 30 cycles of 1.5 min at 94°C, 1.5 min at 65°C and 4 min at 72°C the reaction product was extracted with phenol and phenol chloroform (1:1) and precipitated with ethanol. The DNA was dissolved and after digestion with HindIII and PpumI the 1 Kb fragment was used to replace the HindIII-PpumI fragment of the FGFR2 sequence already cloned in the pBS vector as described above in Example 1. The resulting chimeric FGFR1/KGFR ectodomain was excised from the pBS vector using HindIII and BgIII and subcloned into the same sites of the APtag vector such that it will be expressed as an AP-fusion protein as described in Example 1. The chimeric molecule was also subcloned into M13mp19 and subjected to DNA sequencing by standard chain termination procedures.

To prepare the chimera between FGFR1/KGFR and FGFR2, the second Ig-like domain derived from FGFR1 was replaced by domain 2 of FGFR2 as follows (see Fig. 8): The HindIII-SphI fragment from FGFR1, the AatII-BglII fragment from the FGFR1/KGFR chimera (F1/K in Fig. 8) and the SphI-AatII fragment derived by PCR from FGFR2 (see below) were co-ligated with the APtag vector predigested with HindIII and BglII in a four point ligation. XL1 blue bacteria were then transfected with this vector and clones positive for the above three fragments were analyzed for their structure by DNA sequencing.

The above SphI-AatII fragment was generated by PCR of the FGFR2 in APtag using the following primers:
6) 5'-AAGCGGCTGCATGCTGTCCC corresponding to nucleotides 1144-1163 of FGFR2 and containing an SphI site, and
7) 5'-CCGTTCAACGACGTCGAGGTG, complementary to nucleotides 1187-1407 of FGFR2 and containing an AatII site.

The above restriction sites were introduced by replacing nucleotides (underlined above) without changing the corresponding encoded amino acids. The PCR product was digested with SphI and AatII and included in the above-described four-points ligation. The structure of the so-constructed chimeric receptor is shown schematically in Fig. 8 (F1/F2/K). The designations: D1, D2 and D3 denote the Ig-like domains; AB, the acidic box; SP the signal peptide; and C and V denote the constant and variable regions of Ig domain 3, respectively. The PpumI site represents the beginning of the variable region. HindIII and BglII are the cloning sites in the APtag1 vector, which was employed to prepare AP-fusion protein encoding clones, by the procedure described in Example 1, for FGFR1, KGFR, FGFR1/KGFR and FGFR1/FGFR2/KGFR.

Following the construction of the above recombinant APtag vectors encoding the AP-receptor or AP-chimeric receptor fusion products, NIH 3T3 cells were transfected with calcium phosphate precipitates of each of the APtag recombinant vectors, together with pSV2neo as selective marker for transfection. The resulting G418-resistant cell lines secreting AP activity were selected for further analysis. The cell lines capable of secreting the receptors or chimeric receptors were selected by the determination of the AP activity in the conditioned medium of the transfected cell cultures. The selected cell lines were then cloned and used to assay the ligand binding profile of the secreted soluble receptors and chimeric receptors.

To assay the ligand binding profile of the secreted soluble receptors and chimeric receptors heparin-Sepharose bound ligands were used to bind the receptor-alkalane phosphatase (AP) fusion proteins, the level of which was determined by the AP activity on the conjugated beads. Immobilized ligand was prepared by incubating 300 µl heparin-Sepharose slurry (Pharmacia) with 1 µg of either human recombinant KGF (Amgen), human recombinant bFGF (Takeda, Japan), human recombinant FGF4 (obtained from Pepro-Tech, MA. USA) or human aFGF (obtained from Dr. G. Neufeld, Weizmann Institute, Rehovot, Israel). After 30 min shaking at room temperature, the beads were washed three times with 0.5 M NaCl and suspended (1:1) in 0.01 M phosphate buffer pH 7.4, 0.15 M NaCl (PBS). For receptor-ligand binding profile analysis, 15 µl of ligand-bound heparin-Sepharose beads were incubated with conditioned medium (100-150 µl) from confluent cells secreting FGFR1, KGFR, FGFR1/KGFR or FGFR1/FGFR2/KGFR, normalized for their AP activity. After 30 min of shaking at room temperature the beads were washed three times with 0.5 M NaCl, once with 10 mM Tris-HCl pH 8.0 and incubated at 65°C for 10 min to inactivate endogenous cellular AP. The beads were assayed for AP activity by measuring absorbance at 405 nm after 15 min of incubation with 0.1 M diethanolamine (pH 9.8), 0.5 mM MgCl₂, 10 mM L-homoarginine, 0.5 mg/ml BSA (Sigma) and 12 mM p-nitrophenylphosphate. The AP activity this served as an assay of the extent of binding of the receptors to the immobilized ligand.

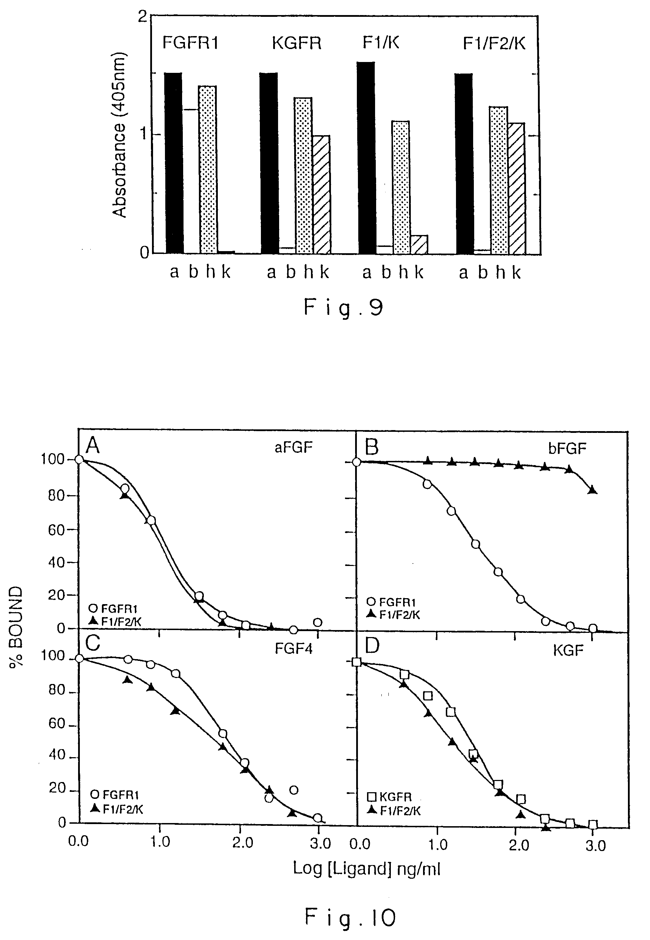

In Fig. 9, the results of the above assay are presented in bargraph form, in which the designations a, b, h and k represent heparin-Sepharose bound aFGF, bFGF, FGF4 and KGF, respectively; and F1/K and F1/F2/K represent the above-noted chimeric receptors. From these results it is apparent that the soluble secreted chimeric receptor FGFR1/KGFR retained essentially the same high binding affinity for aFGF and FGF4 as both parent receptors FGFR1 and KGFR. However, the chimeric FGFR1/KGFR receptor, while losing the ability to bind bFGF, as in the case of the FGFR2/KGFR chimera described in Example 1, did however, unexpectedly not acquire the same ability to bind KGF as in the case of the FGFR2/KGFR chimera. At best, the FGFR1/KGFR chimera acquired a weak KGF binding capability. In contrast, the other chimera FGFR1/FGFR2/KGFR acquired a high KGF binding capability, and overall this chimera possessed a ligand binding profile similar to that of KGFR.

Thus, from the above results it can be concluded that both the Ig domain 2 of either FGFR2 or KGFR and the C-terminal half of Ig domain 3 of KGFR are required in order to endow the FGFR1 with a high KGF binding capability.

The above soluble receptors and chimeric receptors were also analyzed for their binding affinities and specificities, by way of displacement or competition binding analysis procedures. The binding of aFGF, bFGF, FGF4 and KGF to the soluble receptors was analyzed by displacement of labelled FGF4 or KGF with unlabelled FGFs. The reaction mixture (200 µl) contained 0.4 ng of either [¹²⁵I]FGF4 or [¹²⁵I]KGF, increasing amounts of unlabelled ligand, 2 µg heparin (Hepar, Franklin, OH), 0.2% BSA, 25 mM HEPES pH 7.4 and 100 µl of normalized conditioned medium from cells secreting either FGFR1, KGFR, FGFR1/KGFR or FGFR1/FGFR2/KGFR. After 30 min of incubation at room temperature, 15 µl of Sepharose-protein A prebound to rabbit anti-human placental AP antibodies were added and the reaction mixture was shaken for 30 min at room temperature. The beads were washed with HNTG (20 mM HEPES pH 7.5, 150 mM NaCl, 1% Triton X-100 and 10% glycerol) and once with 0.5 M NaCl to eliminate non-specifically bound ligands and the radioactivity associated with the beads was counted in a γ-counter.

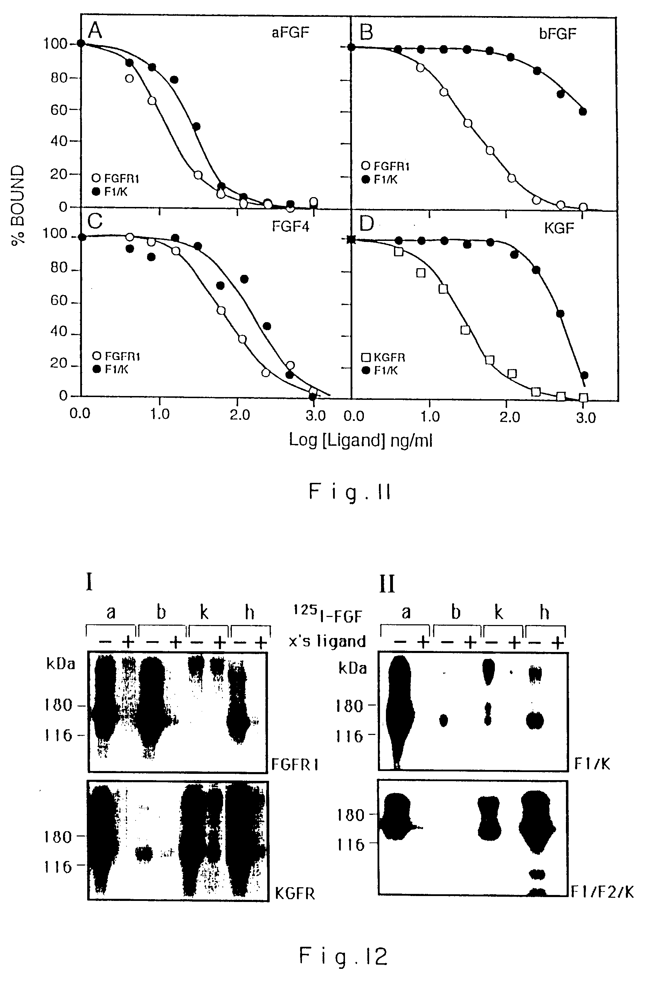

The results of the above displacement binding analysis are presented in graphical form in Figs. 10 and 11. Fig. 11 shows the binding specificities and affinities of the soluble receptors FGFR1 and KGFR and the soluble chimeric receptor FGFR1/KGFR (F1/K), for the ligands aFGF, bFGF, FGF4 and KGF. Panels A, B and C of Fig. 10 show the displacement analysis results of the binding of ¹²⁵I-FGF4 to FGFR1 and F1/K chimera, using unlabelled aFGF (A), bFGF (B) and FGF4 (C). In panel D is shown the results of the displacement analysis of the binding of ¹²⁵I-KGF to KGFR and the F1/K chimera using unlabelled KGF. Fig. 4 shows the binding specificities and affinities of the soluble receptors FGFR1 and KGFR and the soluble chimeric receptor FGFR1/FGFR2/KGFR (F1/F2/K), respectively, for the ligands aFGF, bFGF, FGF4 and KGF. Panels A, B and C of Fig. 4 show the displacement analysis results of the binding of ¹²⁵I-FGF4 to FGFR1 and F1/F2/K chimera, using unlabelled aFGF (A), bFGF (B) and FGF4 (C). Panel D shows the displacement analysis results of the binding of ¹²⁵I-KGF to KGFR and F1/F2/K chimera using unlabelled KGF.

The results presented in Figs. 10 and 11 and summarized in Table 1 below, show that the affinity of aFGF to FGFR1, and to the F1/K chimera is very similar, whereas bFGF does not bind significantly to the F1/K chimera at the tested concentrations. Further, KGF showed approximately 15-fold lower affinity to the FGFR1/KGFR chimera in comparison to the KGFR (IC50 of 500 ng/ml compared to 32 ng/ml, respectively) whereas no measurable binding of KGF to FGFR1 could be demonstrated.

**TABLE 1**

| Comparison of IC₅₀ (nM) for binding of various ligands to soluble FGF receptors | | | | |
|---|---|---|---|---|
| **Receptor** | **Ligand** | | | |
| | **aFGF** | **bFGF** | **FGF4** | **KGF** |
| **FGFR1** | 0.8 | 2 | 4 | >50 |
| **KGFR** | 1.7 | >50 | ND | 1.9 |
| **Fl/K** | 1.7 | >50 | 6.1 | 28 |
| **F1/F2/K** | 0.7 | >50 | 2.8 | 1.0 |

These results also imply that the sequence of the C-terminal half of FGFR1 is essential for bFGF high affinity binding, since its replacement abolished bFGF binding in both FGFR1 and FGFR2 (see Example 1). However, the C-terminal half of Ig domain 3 from KGFR is not sufficient to fully confer on FGFR1 high affinity binding of KGF as it does in the homologous FGFR2/KGFR chimera (see Example 1). In contrast, the FGFR1/FGFR2/KGFR chimera (containing Ig domain 1 and the N-terminal half of Ig domain 3 from FGFR1; Ig domain 2 from FGFR2 or KGFR; and the C-terminal half of Ig domain 3 from KGFR) bind aFGF and FGF4 with high affinity, shows low binding affinity of bFGF, similar to the F1/K chimera, and has a high binding affinity KGF comparable to that of KGFR (Fig. 11 and Table I). These results, also confirm the above results (Fig. 5), namely that both Ig domain 2 and the C-terminal half of Ig domain 3 of KGFR are required in order to confer on FGFR1 high binding affinity towards KGF.

As an additional test, the above secreted, soluble receptors and chimeric receptors were also analyzed with respect to their binding affinities for various ligands by the procedure of chemical cross-linking of soluble secreted receptors and FGFs. Conditioned medium from confluent cells secreting either FGFR1, KGFR, FGFR1/KGFR or FGFR1/FGFR2/KGFR, normalized for AP activity (100 µl) was reacted with either [¹²⁵I]KGF, [¹²⁵I]aFGF, [¹²⁵I]bFGF, or [¹²⁵]FGF4 (10 ng/ml) in the presence of 10 µg/ml heparin and in the absence or presence of a 100-fold excess of unlabelled ligand followed by immunoprecipitation with Sepharose-protein A bound-anti-AP. After extensive washing, the beads were suspended in 0.4 ml of 0.15 mM disuccinylimidyl suberate (DSS, Pierce) in PBS for 30 min at room temperature, to effect the cross-linking of ligand to receptor, and then spun and re-suspended in 1 ml of 150 mM glycine in 10 mM Tris-HCl pH 7.5 to terminate the cross-linking reaction. After 5 minutes the beads were washed with PBS, boiled for 5 min in sample buffer and the proteins were analyzed by PAGE on a 6% gel. After the run, the gel was dried and exposed to an X-ray film for 16 h.

Reproductions of the exposed x-ray film from the above chemical cross-linking affinity labelling analysis of the various FGFRs and chimeric FGFRs are presented in Fig. 12. Panel I of Fig. 12 shows the results for soluble receptors FGFR1 and KGFR, and in panel II, the results for the soluble chimeric receptors FGFR1/KGFR and FGFR1/FGFR2/KGFR, (F1/K and F1/F2/K, respectively). In both panels I and II, the designations a, b, h, and k denote the ¹²⁵I-labelled ligands aFGF, bFGF, FGF4 and KGF, respectively, whilst "x's ligand" and the "-" or "+" sign used in connection therewith denotes the presence (+) or absence (-) of a 100-fold excess of the same unlabelled ligand. In both panels the numbers at the left side thereof denote the relative positions of molecular weight markers (molecular weight in kDa) run on the same gel under the same conditions.

The results in Fig. 12 demonstrate that the ligand bound receptor migrate as two major bands which very likely represent monomer and dimer forms of receptor-ligand complexes. The F1/K receptor chimera was strongly labelled with either aFGF or FGF4. The labelling with KGF was significantly weak and seemed to be associated with the appearance of some dimeric receptors as well. The parent receptor FGFR1 was not labelled at all with KGF consistent with the qualitative and quantitative ligand binding profiles (see above). In contrast, the chimeric receptor F1/F2/K showed high labelling with KGF which was specifically inhibited by unlabelled KGF. These results are in complete agreement with both the binding data (Figs. 10 a 11) and the ligand binding profile (Fig. 9).

Hence, the results show, by three independent methods, that the chimera F1/F2/K gained high affinity binding of KGF, this binding affinity being determined by sequence determinants present in both domain 2 of either FGFR2 or KGFR origin, and the C-terminal of domain 3, of KGFR origin.

In view of all of the results presented hereinabove in Examples 1 and 2 and their accompanying figures it is possible to summarize as follows:
The extracellular domain of FGF receptors is organized as three immunoglobulin-like domains of which the two closest to the membrane contain all the necessary elements for high affinity binding of FGF. The results presented hereinabove, demonstrate that the variable sequences in domain 3 can differentially affect high affinity binding of different members of the FGF family. The present results also demonstrate that multiple non-contiguous elements which are contained in domain 2 and domain 3 participate in the ligand-binding are only the binding specificity of FGFRs.

Based on the above-described findings one can construct more chimeric receptors with different ligand binding specificities. Moreover, inserting mutations within the variable region of Ig-domain 3 and/or domain 2 will allow one to modulate the binding activities of the various receptors. These can serve as the basis for the construction of dominant negative mutations which can be used for future gene therapy as specified repressors of both the mitogenic and transforming activities of FGFs *in vivo*. The invention can also serve as a basis for developing new therapeutic agents for modulating growth factor-receptor interactions both *in vitro* and *in vivo*, e.g. use of a BSR derived polypeptide as a competitive inhibitor of the FGFR stimulating pathway.

## Claims

1. A polypeptide being a member selected from the group consisting of:
(a) a polypeptide having an amino acid sequence of the ligand specificity region (LSR) from the extracellular portion of an FGFR, which region confers the ligand binding specificity of said FGFR;
(b) a polypeptide having the amino acid sequence of the polypeptide of (a) in which one or more amino acids residues has been added, replaced or deleted, which polypeptide when inserted into an extracellular portion of an FGFR in place of said LSR, confer upon said FGFR the same ligand binding specificity of said LSR; and
(c) a recombinant polypeptide or protein having an amino acid sequence comprising the amino acid sequence of (a) or (b).

2. A polypeptide according to Claim 1, as defined under (a), (b) or (c), wherein said LSR is included in the C-terminal region of immunoglobulin like domain 3 of the FGFR.

3. A polypeptide according to Claim 2, wherein said LSR is included in said C-terminal region of an FGFR selected from the group consisting of murine FGFR1, murine FGFR2, murine KGFR, human K-Sam, and of FGFRs from other mammals which are homologous to the aforementioned.

4. A polypeptide according to Claim 3, wherein said LSR is included in the amino acid sequence depicted in Figs. 1, 3 or 4 or in a homologous sequence from another mammal.

5. A polypeptide according to Claim 1, wherein said LSR is included in the immunoglobulin like domain 2 of the FGFR.

6. A polypeptide according to Claim 5, wherein said LSR is included in said domain 2 of an FGFR selected from the goup consisting of the murine FGFR1, FGFR2 and KGFR and of FGFRs from other mammals which are homologous to the aforementioned.

7. A chimeric protein comprising one or more first regions derived from a first protein and one or more second regions derived from a second protein, said first and second regions being fused to one another; the protein being characterized in that:
(i) said one or more first regions being each independently a member selected from the group consisting of -
(a) a polypeptide chain having an amino acid sequence of the ligand specificity region (LSR) from the extracellular portion of a first fibroblast growth factor receptor (FGFR), which region comfers the ligand binding specificity of said FGFR, and
(b) a polypeptide chain having the ligand specificity properties of said LSR and having the amino acid sequence of the polypeptide in (a) in which one or more amino acid residues has been added, replaced or deleted;
(ii) said one or more second regions being each independently a member selected from the group consisting of -
(a) a polypeptide chain having an amino acid sequence of a region of a second FGFR which is not the LSR (non-LSR) of said second FGFR, and
(b) a polypeptide chain having the amino acid sequence of said non-LSR in which one or more amino acid residues has been replaced or added without essentially affecting the biological properties of said second region or the binding specificity of said LSR; and in that
(iii) said chimeric protein being capable of binding a ligand and having a ligand binding specificity conferred by said one or more first regions.

8. A chimeric protein according to Claim 7, wherein said first and second FGFR are selected from the group consisting of the murine FGFR1, murine FGFR2, murine KGFR, the human K-Sam, and of homologous FGFRs from other mammals.

9. A chimeric protein according to Claim 7, comprising an extracellular portion of one FGFR wherein the LSR in the C-terminal region of its immunoglobulin-like domain 3 has been replaced with a corresponding LSR from another FGFR.

10. A chimeric protein according to Claim 9, wherein said LSR is included in the amino acid sequence depicted in Figs. 1, 2 or 4 or in an homologous sequence from another mammal.

11. A chimeric protein according to Claim 7, comprising an extracellular portion of one FGFR in which the LSR from immunoglobulin like domain 2 and the LSR from the C-terminal of the immunoglobulin like domain 3 are replaced by corresponding LSRs from other FGFR, the two LSRs coming both from the same or from a different other FGFR.

12. A DNA molecule comprising a coding sequence being a member selected from the group consisting of:
(a) a nucleotide sequence encoding the ligand specificity region (LSR) from the extracellular portion of an FGFR, which region confers the ligand binding specificity of said FGFR;
(b) a nucleotide sequence of (a) in which one or more codon has been added, replaced or deleted, provided that the encoded polypeptide retains the ligand specificity properties of said LSR; and
(c) a recombinant nucleotide sequence encoding a polypeptide and comprising the sequence of (a) or (b).

13. A DNA molecule according to Claim 12, as defined under (a), (b) or (c), wherein said LSR is included in a C-terminal region of an immunoglobulin domain 3 of an FGFR.

14. A DNA molecule according to Claim 13, wherein said LSR is obtained from an FGFR selected from the group consisting of the murine FGFR1, murine FGFR2, murine KGFR, human K-Sam, and of a homologous FGFR from another mammal.

15. A DNA molecule according to Claim 14, wherein said LSR is included in the amino acid sequence depicted in Figs. 1, 2 and 4 or in an homologous amino acid sequence from another mammal.

16. A DNA molecule according to Claim 12, as defined under (a), (b) or (c), wherein said LSR is included in the immunoglobulin like domain 2 of an FGFR.

17. A DNA molecule according to Claim 16, wherein said LSR is obtained from an FGFR, selected from the group consisting of murine FGFR1, murine FGFR2, murine KGFR and of a homologous FGFR from another mammal.

18. A DNA molecule according to Claim 12, encoding a chimeric protein comprising a ligand specificity region (LSR) derived from a first FGFR and a non-LSR derived from a second FGFR.

19. An expression vector comprising the DNA molecule of Claim 12.

20. A cell transfected by the vector of Claim 19.

21. An expression product of the cell of Claim 20.
